# EUROPÄISCHE PATENTSCHRIFT

(11) **EP 3 359 525 B1**
(45) Veröffentlichungstag und Bekanntmachung des Hinweises auf die Patenterteilung: **06.01.2021**
(21) Anmeldenummer: 16787350.4
(22) Anmeldetag: 04.10.2016
(51) Int. Cl.: C07D 209/54, A01N 43/38, A01P 13/00

(54) **NEUE ALKINYL-SUBSTITUIERTE 3-PHENYLPYRROLIDIN-2,4-DIONE UND DEREN VERWENDUNG ALS HERBIZIDE**
NEW ALKYNYL-SUBSTITUTED 3-PHENYLPYRROLIDIN-2,4-DIONES AND THEIR USE AS HERBICIDES
NOUVELLES 3-PHÉNYLPYRROLIDIN-2,4-DIONES SUBSTITUTÉES PAR UN GROUPEMENT ALKYNYLE ET LEUR UTILISATION EN TANT QU'HERBICIDES

(30) Priorität: 06.10.2015 EP 15188613
(43) Veröffentlichungstag der Anmeldung: 15.08.2018
(60) Teilanmeldung: 20196445.9
(73) Patentinhaber: Bayer CropScience Aktiengesellschaft, 40789 Monheim am Rhein (DE)
(72) Erfinder: ANGERMANN, Alfred, 65830 Kriftel (DE); LEHR, Stefan, 65835 Liederbach (DE); FISCHER, Reiner, 40789 Monheim (DE); BOJACK, Guido, 65207 Wiesbaden-Naurod (DE); HELMKE, Hendrik, 65835 Liederbach (DE); SCHMUTZLER, Dirk, 65795 Hattersheim (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim (DE)
(74) Vertreter: BIP Patents
(86) Internationale Anmeldenummer: PCT/EP2016/073590
(87) Internationale Veröffentlichungsnummer: WO 2017/060203

(56) Entgegenhaltungen:
- WO-A1-01/74770
- WO-A1-2015/040114

## Beschreibung

Die vorliegende Erfindung betrifft neue herbizid wirksame Alkinyl-substituierte 3-Phenylpyrrolidin-2,4-dione gemäß der allgemeinen Formel (I) oder agrochemisch akzeptable Salze davon, sowie deren Verwendung zur Bekämpfung von Unkräutern und Ungräsern in Nutzpflanzenkulturen.

Die Verbindungsklasse der 3-Arylpyrrolidin-2,4-dione sowie deren Herstellung und Verwendung als Herbizide sind aus dem Stand der Technik wohl bekannt. Darüber hinaus sind auch bicyclische 3-Aryl-pyrrolidin-2,4-dion-Derivate (EP-A-355 599, EP-A-415 211 und JP-A-12-053 670 ff.) sowie substituierte monocyclische 3-Aryl-pyrrolidin-2,4-dion-Derivate (EP-A-377 893 und EP-A-442 077 ff.) mit herbizider, insektizider oder fungizider Wirkung beschrieben.

Alkinyl-substituierte-3-Phenylpyrrolidin-2,4-dione mit herbizider Wirkung sind ferner aus WO 96/82395, WO 98/05638, WO 01/74770, WO 14/032702 oder WO15/040114 bekannt.

Die Wirksamkeit dieser Herbizide gegen Schadpflanzen ist von zahlreichen Parametern abhängig, beispielsweise von der verwendeten Aufwandmenge, der Zubereitungsform (Formulierung), den jeweils zu bekämpfenden Schadpflanzen, dem Schadpflanzenspektrum, den Klima- und Bodenverhältnissen sowie der Dauer der Wirkung bzw. der Abbaugeschwindigkeit des Herbizids. Zahlreiche Herbizide aus der Gruppe der 3-Arylpyrrolidin-2,4-dione erfordern, um eine ausreichende herbizide Wirkung zu entfalten, hohe Aufwandmengen und/oder schmale Schadpflanzenspektren, was deren Anwendung ökonomisch unattraktiv macht. Es besteht daher der Bedarf an alternativen Herbiziden, die verbesserte Eigenschaften aufweisen sowie ökonomisch attraktiv und gleichzeitig effizient sind. Aufgabe der vorliegenden Erfindung ist folglich die Bereitstellung von neuen Verbindungen, die die genannten Nachteile nicht aufweisen.

Die vorliegende Erfindung betrifft daher neue Alkinyl-substituierte-N-Phenylpyrrolidin-2,4-dione_der allgemeinen Formel (I), 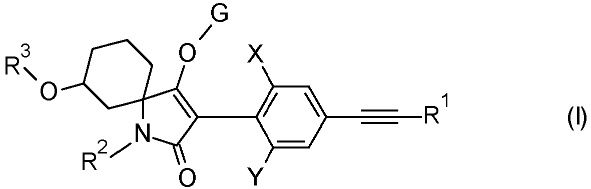 oder ein agrochemisch akzeptables Salz davon,
wobei
- X: = C₁-C₄-Alkyl, C₁-C₄-Haloalkyl oder C₃-C₆-Cycloalkyl,
- Y: = C₁-C₄-Alkyl oder C₃-C₆-Cycloalkyl,
- R¹: = Wasserstoff, C₁-C₆-Alkyl, oder C₃-C₆-Cycloalkyl,
- R²: = Wasserstoff oder Methyl,
- R³: = C₁-C₆-Alkyl oder C₁-C₆-Alkoxy- C₂-C₆-alkyl,
- G: = Wasserstoff, eine abspaltbare Gruppe L oder ein Kation E; wobei L = einer der folgenden Reste 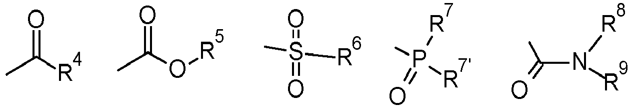
worin
R⁴ = C₁-C₄-Alkyl oder C₁-C₃-Alkoxy-C₁-C₄-Alkyl,
R⁵ = C₁-C₄-Alkyl,
R⁶ = C₁-C₄-Alkyl, ein unsubstituiertes Phenyl oder ein einfach oder mehrfach mit Halogen, C₁-C₄-Alkyl, C₁-C₄-Haloalkyl, C₁-C₄-Alkoxy, C₁-C₄-Haloalkoxy, Nitro oder Cyano substituiertes Phenyl,
R⁷, R^{7'} = unabhängig voneinander Methoxy oder Ethoxy,
R⁸ und R⁹ = jeweils unabhängig voneinander Methyl, Ethyl, Phenyl oder gemeinsam einen gesättigten 5-, 6- oder 7-gliedrigen Ring bilden, oder gemeinsam einen gesättigten 5-, 6-, oder 7-gliedrigen Heterozyklus mit einem Sauerstoff- oder Schwefelatom bilden,
E = ein Alkalimetallion, ein Ionenäquivalent eines Erdalkalimetalls, ein Ionenäquivalent Aluminium, ein Ionenäquivalent eines Übergangsmetalls, ein Magnesium-Halogen-Kation, oder
   ein Ammoniumion, bei dem gegebenenfalls ein, zwei, drei oder alle vier Wasserstoffatome durch gleiche oder verschiedene Reste aus den Gruppen Wasserstoff, C₁-C₅-Alkyl, C₁-C₅-Alkoxy oder C₃-C₇-Cycloalkyl, die jeweils ein- oder mehrfach mit Fluor, Chlor, Brom, Cyano, Hydroxy substituiert oder durch ein- oder mehrere Sauerstoff- oder Schwefelatome unterbrochen sein können, oder
   ein cyclisches sekundäres oder tertiäres aliphatisches oder heteroaliphatisches Ammoniumion, beispielsweise Morpholinium, Thiomorpholinium, Piperidinium, Pyrrolidinium, oder jeweils protoniertes 1,4-Diazabicyclo[1.1.2]octane (DABCO) oder 1,5-Diazabicyclo[4.3.0]undec-7-en (DBU), oder
   ein heterocyclisches Ammoniumkation, beispielsweise jeweils protoniertes Pyridin, 2-Methylpyridin, 3-Methylpyridin, 4-Methylpyridin, 2,4-Dimethylpyridin, 2,5-Di-methylpyridin, 2,6-Dimethylpyridin, 5-Ethyl-2-methylpyridin, Pyrrol, Imidazol, Chinolin, Chinoxalin, 1,2-Dimethylimidazol, 1,3-Dimethylimidazolium-methylsulfat, steht, oder weiterhin für ein Sulfoniumion.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle wie n-Pentyl, 2,2,-Dimethylpropyl und 3-Methylbutyl. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl.
Halogen bedeutet Fluor, Chlor, Brom oder Iod.

Die Verbindungen der Formel (I) können, in Abhängigkeit von der Art der Substituenten, als geometrische und/oder optische Isomere oder Isomerengemische, in unterschiedlicher Zusammensetzung vorliegen, beispielsweise auch in cis- oder trans-Form, welche folgendermaßen definiert sind:

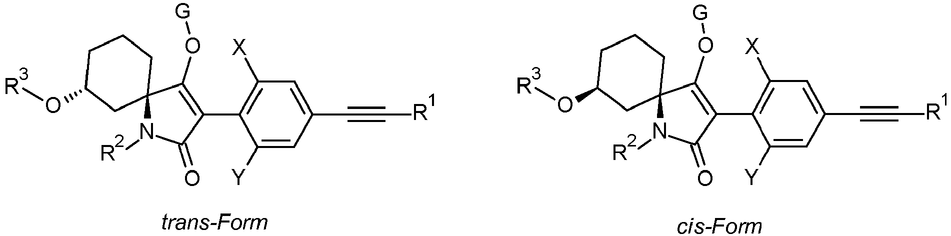

Die gegebenfalls bei der Synthese anfallenden Isomerengemische können mit den üblichen technischen Methoden getrennt werden.

Sowohl die reinen Isomeren als auch die Tautomeren- und Isomerengemische, deren Herstellung und Verwendung sowie diese enthaltende Mittel sind Gegenstand der vorliegenden Erfindung. Im Folgenden wird der Einfachheit halber jedoch stets von Verbindungen der Formel (I) gesprochen, obwohl sowohl die reinen Verbindungen als auch gegebenenfalls Gemische mit unterschiedlichen Anteilen an isomeren und tautomeren Verbindungen gemeint sind.

Bevorzugt sind Verbindungen, in denen
- X: = C₁-C₄-Alkyl oder C₃-C₆-Cycloalkyl,
- Y: = C₁-C₄-Alkyl oder C₃-C₆-Cycloalkyl,
- R¹: = Wasserstoff, Methyl, Ethyl, Isopropyl oder Cyclopropyl,
- R²: = Wasserstoff oder Methyl,
- R³: = C₁-C₆-Alkyl oder C₁-C₄-Alkoxy-C₂-C₄-alkyl,
- G: = Wasserstoff, eine abspaltbarc Gruppe L oder ein Kation E worin
L = einer der folgenden Reste 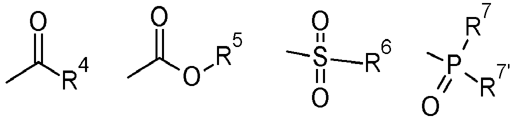
worin
- R⁴: = C₁-C₄-Alkyl,
- R⁵: = C₁-C₄-Alkyl,
- R⁶: = C₁-C₄-Alkyl, ein unsubstituiertes Phenyl oder ein mit Halogen, C₁-C₄-Alkyl oder C₁-C₄-Alkoxy substituiertes Phenyl,
R⁷, R^{7'} = unabhängig voneinander Methoxy oder Ethoxy,
- E: = ein Alkalimetallion, ein Ionenäquivalent eines Erdalkalimetalls, ein Ionenäquivalent Aluminium oder ein Ionenäquivalent eines Übergangsmetalls, oder
ein Ammoniumion, bei dem gegebenenfalls ein, zwei, drei oder alle vier Wasserstoffatome durch gleiche oder verschiedene Reste aus den Gruppen Wasserstoff oder C₁-C₅-Alkyl, oder ein tertiäres aliphatisches oder heteroaliphatisches Ammoniumion, oder ein heterocyclisches Ammoniumkation, beispielsweise jeweils protoniertes Pyridin, Chinolin, Chinoxalin, 1,2-Dimethylimidazol, 1,3-Dimethylimidazolium-methylsulfat, steht, oder weiterhin für ein Sulfoniumion.

Besonders bevorzugt sind Verbindungen der allgemeinen Formel (I), in denen
- X: = Methyl, Ethyl oder Cyclopropyl,
- Y: = Methyl oder Ethyl,
- R¹: = Wasserstoff, Methyl, Ethyl, Isopropyl oder Cyclopropyl,
- R²: = Wasserstoff,
- R³: = C₁-C₄-Alkyl oder C₁-C₃-Alkoxy-C₂-C₄-alkyl,
- G: = Wasserstoff, eine abspaltbare Gruppe L oder ein Kation E worin
L = einer der folgenden Reste 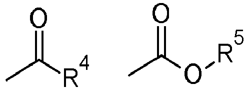
worin
R⁴ = C₁-C₄-Alkyl,
R⁵ = C₁-C₄-Alkyl,
E = ein Alkalimetallion, ein Ionenäquivalent eines Erdalkalimetalls, ein Ionenäquivalent Aluminium, ein Ionenäquivalent eines Übergangsmetalls oder für ein Magnesium-Halogen-Kation, ein Tetra-C₁-C₅-Alkylammoniumkation oder ein heterocyclisches Ammoniumkation, beispielsweise jeweils protoniertes Pyridin oder Chinolin, steht.

Ganz besonders bevorzugt sind Verbindungen der Formel (I), in denen
- X: = Methyl oder Ethyl
- Y: = Methyl oder Ethyl,
- R¹: = Wasserstoff, Methyl, Ethyl oder Cyclopropyl,
- R²: = Wasserstoff,
- R³: = C₁-C₄-Alkyl oder C₁-C₃-Alkoxy-C₂-C₄-alkyl,
- G: = Wasserstoff, eine abspaltbare Gruppe L oder ein Kation E worin
L = einer der folgenden Reste 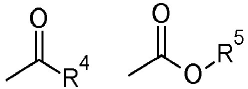
worin
- R⁴: = Methyl, Ethyl oder Isopropyl,
- R⁵: = Methyl, Ethyl oder Isopropyl,
- E: = ein Natrium-, Kalium-, Trimethylammonium-, Pyridinium-, Chinolinium- oder Trimethylsulfonium-Kation oder ein Ionenäquivalent Calcium- oder Magnesium
bedeuten.

Im Einzelnen seien zur Illustration folgende erfindungsgmäßen Verbindungen genannt:

**Tabelle 1: Beispiel-Nummern 1.01-1.42 mit R¹ = H**

| | | | |
|---|---|---|---|
| 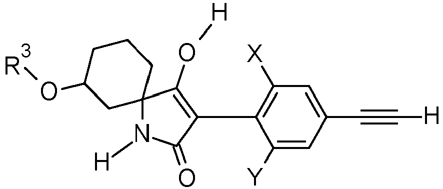 | | | |

| **Beispiel Nr.** | **R³** | **X** | **Y** |
|---|---|---|---|
| 1.01 | Me | H | Me |
| 1.02 | Me | H | Et |
| 1.03 | Me | Me | Me |
| 1.04 | Me | Me | Et |
| 1.05 | Me | Et | Et |
| 1.06 | Me | Cyclopropyl | Me |
| 1.07 | Me | Cyclopropyl | Et |
| 1.08 | Et | H | Me |
| 1.09 | Et | H | Et |
| 1.10 | Et | Me | Me |
| 1.11 | Et | Me | Et |
| 1.12 | Et | Et | Et |
| 1.13 | Et | Cyclopropyl | Me |
| 1.14 | Et | Cyclopropyl | Et |
| 1.15 | n-Propyl | H | Me |
| 1.16 | n-Propyl | H | Et |
| 1.17 | n-Propyl | Me | Me |
| 1.18 | n-Propyl | Me | Et |
| 1.19 | n-Propyl | Et | Et |
| 1.20 | n-Propyl | Cyclopropyl | Me |
| 1.21 | n-Propyl | Cyclopropyl | Et |
| 1.22 | i-Propyl | H | Me |
| 1.23 | i-Propyl | H | Et |
| 1.24 | i-Propyl | Me | Me |
| 1.25 | i-Propyl | Me | Et |
| 1.26 | i-Propyl | Et | Et |
| 1.27 | i-Propyl | Cyclopropyl | Me |
| 1.28 | i-Propyl | Cyclopropyl | Et |
| 1.29 | n-Butyl | H | Me |
| 1.30 | n-Butyl | H | Et |
| 1.31 | n-Butyl | Me | Me |
| 1.32 | n-Butyl | Me | Et |
| 1.33 | n-Butyl | Et | Et |
| 1.34 | n-Butyl | Cyclopropyl | Me |
| 1.35 | n-Butyl | Cyclopropyl | Et |
| 1.36 | CH₃OCH₂CH₂O- | H | Me |
| 1.37 | CH₃OCH₂CH₂O- | H | Et |
| 1.38 | CH₃OCH₂CH₂O- | Me | Me |
| 1.39 | CH₃OCH₂CH₂O- | Me | Et |
| 1.40 | CH₃OCH₂CH₂O- | Et | Et |
| 1.41 | CH₃OCH₂CH₂O- | Cyclopropyl | Me |
| 1.42 | CH₃OCH₂CH₂O- | Cyclopropyl | Et |

Die obengenannten Verbindungen 1.08, 1.09,1.15,1.16,1.22,1.23,1.29,1.30,1.36 und 1.37 fallen nicht unter die Ansprüche und stellen somit Referenzverbindungen dar.

### Tabelle 2: Beispiel-Nummern 2.01-2.42 mit R¹ = CH₃

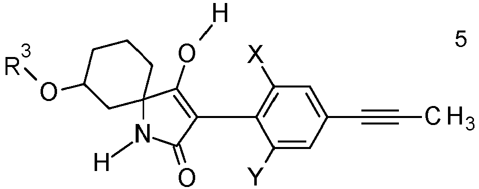

Beispiel-Nummern 2.01 - 2.42, wobei R³, X und Y identisch mit denen in Tabelle 1 sind Die obengenannten Verbindungen 2.08, 2.09,2.15,2.16,2.22,2.23,2.29,2.30,2.36 und 2.37 fallen nicht unter die Ansprüche und stellen somit Referenzverbindungen dar.

### Tabelle 3: Beispiel-Nummern 3.01-3.42 mit R¹ = C₂H₅

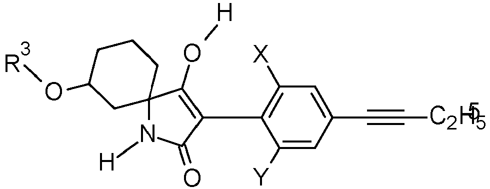

Beispiel-Nummern 3.01 - 3.42, wobei R³, X und Y identisch mit denen in Tabelle 1 sind Die obengenannten Verbindungen 3.08, 3.09,3.15,3.16,3.22,3.23,3.29,3.30,3.36 und 3.37 fallen nicht unter die Ansprüche und stellen somit Referenzverbindungen dar.

### Tabelle 4: Beispiel-Nummern 4.01-4.42 mit R¹ = nC₃H₇

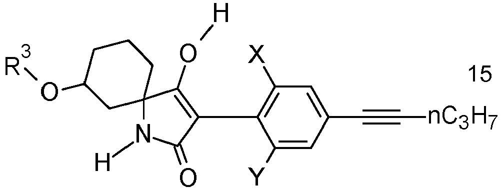

Beispiel-Nummern 4.01 - 4.42, wobei R³, X und Y identisch mit denen in Tabelle 1 sind Die obengenannten Verbindungen 4.08, 4.09,4.15,4.16,4.22,4.23,4.29,4.30,4.36 und 4.37 fallen nicht unter die Ansprüche und stellen somit Referenzverbindungen dar.

### Tabelle 5: Beispiel-Nummern 5.01-5.42 mit R¹ = Isopropyl

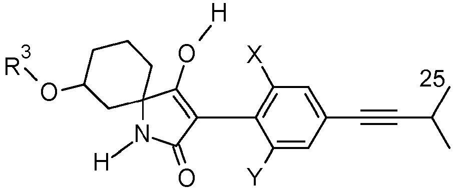

Beispiel-Nummern 5.01-5.42, wobei R³, X und Y identisch mit denen in Tabelle 1 sind Die obengenannten Verbindungen 5.08, 5.09,5.15,5.16,5.22,5.23,5.29,5.30,5.36 und 5.37 fallen nicht unter die Ansprüche und stellen somit Referenzverbindungen dar.

### Tabelle 6: Beispiel-Nummern 6.01-6.42 mit R¹ = Cyclopropyl

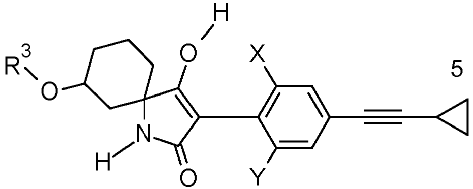

Beispiel-Nummern 6.01-6.42, wobei R³, X und Y identisch mit denen in Tabelle 1 sind Die obengenannten Verbindungen 6.08, 6.09,6.15,6.16,6.22,6.23,6.29,6.30,6.36 und 6.37 fallen nicht unter die Ansprüche und stellen somit Referenzverbindungen dar.

Die Herstellung der erfindungsgemäßen Verbindungen der allgemeinen Formel (I) kann in Anlehnung an literaturbekannte Verfahren erfolgen, beispielsweise indem man
a) eine Verbindung der allgemeinen Formel (II) 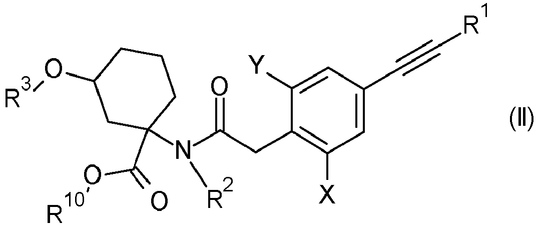 in welcher X, Y, R¹, R² und R³ die oben angegebenen Bedeutungen haben, und R¹⁰ für Alkyl, bevorzugt für Methyl oder Ethyl steht, gegebenenfalls in Anwesenheit eines geeigneten Lösungs- oder Verdünnungsmittels, mit einer geeigneten Base unter formaler Abspaltung der Gruppe R¹⁰OH cyclisiert, oder
b) eine Verbindung der allgemeinen Formel (Ia), 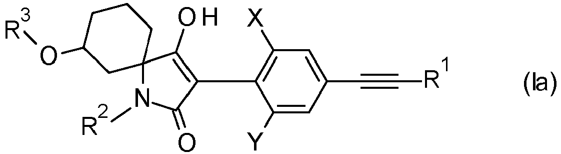 in der X, Y, R¹, R² und R³ die oben angegebenen Bedeutungen haben, mit einer Verbindung der allgemeinen Formel (III),

   Hal-L (III)

   in der L die oben angegebene Bedeutung hat und Hal für ein Halogen, vorzugsweise Chlor oder Brom oder eine Sulfonsäuregruppe stehen kann, gegebenenfalls in Anwesenheit eines geeigneten Lösungs- oder Verdünnungsmittels sowie einer geeigneten Base, zur Reaktion bringt,
(c) indem man Verbindungen der allgemeinen Formel (IV), 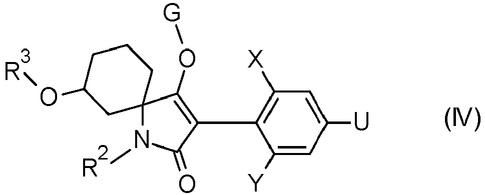 in der X, Y, R² und R³ und G die oben angegebenen Bedeutungen haben, und U für eine geeignete Abgangsgruppe wie beispielsweise Brom, Iod, Triflat oder Nonaflat steht, mit einem geeigneten Alkinyl-Reagenz der allgemeinen Formel (V), 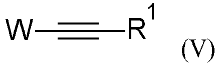 in der R¹ die oben angegebene Bedeutung besitzt und W für Wasserstoff oder eine geeignete Abgangsgruppe steht, gegebenenfalls in Gegenwart geeigneter Katalysatoren und einer geeigneten Base, umsetzt. Als Abgangsgruppe W kommen beispielsweise Halogenatome wie Chlor, Brom oder Iod, Alkylsulfonestergruppen wie beispielsweise Triflat, Mesylat oder Nonaflat, Magnesiumchlorid, Zinkchlorid, ein Trialkylzinnrest, Carboxyl sowie Borsäure-Reste wie -B(OH)₂ oder -B(OAlkyl)₂ in Betracht. Als Katalysatoren sind insbesondere Pd⁰ Komplexe sehr gut geeignet, wobei in vielen Fällen auch der Zusatz von Cu^{(I)}-Salzen sehr vorteilhaft sein kann.

Die beschriebene Methodik ist Stand der Technik und im Übrigen auch unter dem Stichwort "Palladium-katalysierte Kreuzkupplung", "Sonogashira-, Negishi-, Suzuki-, Stille- oder Kumada-Kupplung" einschlägig literaturbekannt.

Alternativ kann eine Verbindung der allgemein Formel (IV) auch mit einem Alkinyl-Reagenz der allgemeinen Formel (VI) in analoger Anwendung der oben beschriebenen Kupplungs-Methodik umsetzt werden, anschließend in Ethinyl-Verbindungen der allgemeinen Formel (VIII) gespalten und diese schließlich mit einem geeigneten Alkylierungsreagenz in die erfindungsgemäße Verbindung (I) überführt werden, wobei jeweils X, Y, R¹, R², R³, G, U und W die beschriebene Bedeutung haben und die abspaltbare Gruppe R¹¹ beispielsweise für eine Gruppe (C₁-C₄-Alkyl)₂C-OH oder auch Trimethylsilyl stehen kann.

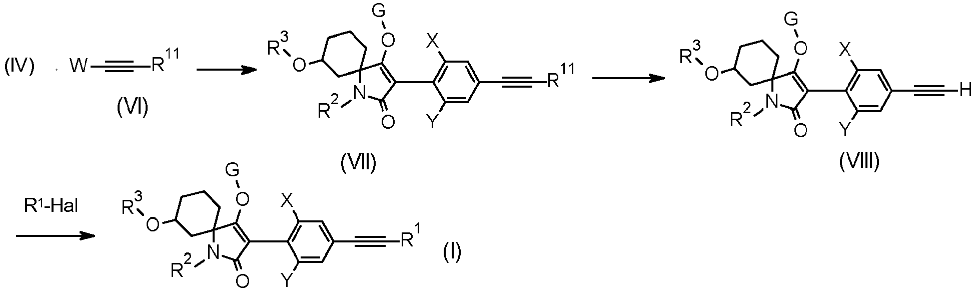

Diese ebenfalls literaturbekannte Technik ist beispielsweise in Beilstein Journal of Organic Chemistry 2011, 7(55), 426-431 und Catalysis Communications 2015, 60, 82-87 näher erläutert.

Für den Fall, daß der Rest R¹ in der allgemeinen Formel (I) für Methyl steht und X, Y, R², R³ sowie G, U und W die weiter oben beschriebene Bedeutung besitzen, besteht eine weitere Alternative darin, eine Verbindung der allgemeinen Formel (IV) mit einem Alkinyl-Reagenz der allgemeinen Formel (IX), worin R¹² beispielsweise für einen C₁-C₄-Trialkylsilylrest steht und W die oben angegebene Bedeutung besitzt, in analoger Anwendung der oben beschriebenen Kupplungs-Methodik zu einer Verbindung der allgemeinen Formel (X) umzusetzen. Die Gruppe R¹² kann anschließend unter geeigneten Bedingungen abgespalten werden und man erhält erfindungsgemäße Verbindungen der Formel (I) mit R³ = Me.

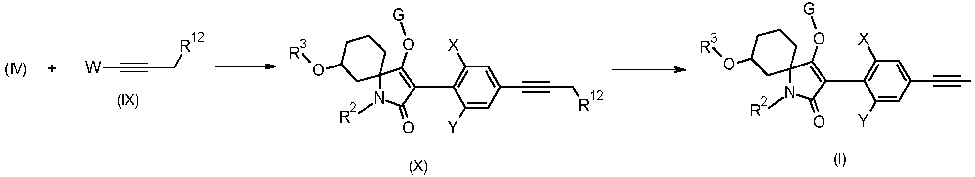

Diese literaturbekannte Technik ist beispielsweise im Journal of Medicinal Chemistry 2007, 50 (7), 1627-1634 beschrieben.

Die benötigten Vorstufen der allgemeinen Formel (II) 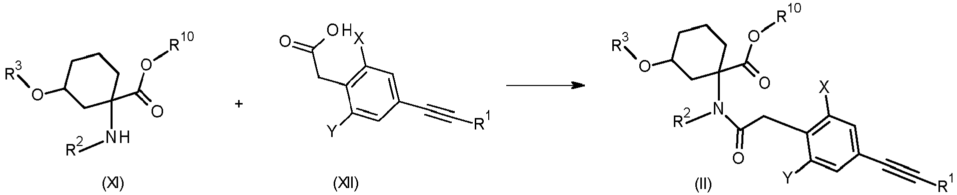 können in Analogie zu bekannten Verfahren, beispielsweise durch Umsetzung eines Aminosäureesters der allgemeinen Formel (XI) mit einer Phenylessigsäure der allgemeinen Formel (XII), worin X, Y, R¹, R² und R³ und R¹⁰ die oben beschriebene Bedeutung haben, gegebenenfalls durch Zusatz eines wasserentziehenden Mittels und gegebenenfalls in Anwesenheit eines geeigneten Lösungs- bzw. Verdünnungsmittels, hergestellt werden.

Eine weitere Variante zur Herstellung von Vorstufen der allgemeinen Formel (II) besteht unter anderem auch darin, dass man eine Verbindung mit der allgemeinen Formel (XIII), in der X, Y, R², R³, R¹⁰ und U die oben angegebene Bedeutung haben, nach der bereits beschriebenen Kreuzkupplungs-Methodik mit einer Verbindung der allgemeinen Formel (V) oder (VI), in der W, R³ und R¹¹ die oben angegebene Bedeutung haben, umsetzt:

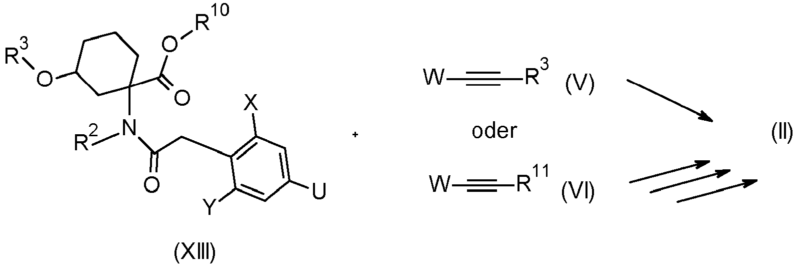

Phenylessigsäuren der allgemeinen Formel (VII) - namentlich 2,6-Dimethyl-4-propargylphenylessigsäure - sind in WO 2015/040114 prinzipiell erwähnt, jedoch wird kein Zugangsweg zu diesen Verbindungen beschrieben.

Sie lassen sich jedoch in Anlehnung an literaturbekannte Verfahren herstellen, beispielsweise indem man eine Verbindung der allgemeinen Formel (X), wobei X, Y, U, wie oben definiert sind und R = C₁-C₄-Alkyl, wiederum mit weiter oben bereits beschriebenen Technik mit Reagenzien der allgemeinen Formel (V) oder (V), wobei W, R¹ und R¹¹ wie oben definiert sind, umsetzt.

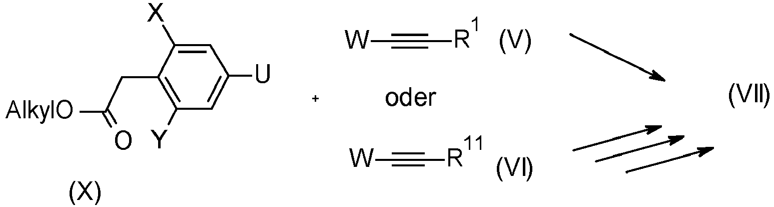

Die erfindungsgemäßen Verbindungen der Formel (I) und/oder deren Salze, im Folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg, oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 1. Aufl. Cold Spring Harbor Laboratory Press, Cold Spring Harbor, NY, oder Winnacker "Gene und Klone", VCH Weinheim 1. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

In einer bevorzugten Ausführungsform der vorliegenden Erfindung können mit den Verbindungen der allgemeinen Formel (I) auch solche Schadpflanzen, z.B. aus der Gruppe Agrostis, Alopecurus, Apera, Avena, Brachiaria, Bromus, Cenchrus, Digitaria, Echinochloa, Eleusine, Eriochloa, Leptochloa, Lolium, Ottochloa, Panicum, Pennisetum, Phalaris, Poa, Rottboellia, Setaria und/oder Sorghum Unkräuter; insbesondere Alopecurus, Apera, Avena, Brachiaria, Bromus, Digitaria, Echinochloa, Eriochloa, Lolium, Panicum, Phalaris, Poa, Setaria und/oder Sorghum Unkräuter bekämpft werden,
- die gegenüber einem oder mehreren das Enzym Acetyl-CoA-Carboxylase (ACCase) inhibierenden Herbiziden resistent sind. ACCase-inhibierende Herbizide sind u.a. Pinoxaden, Clodinafop-propargyl, Fenoxaprop-P-ethyl, Diclofop-methyl, Fluazifop-P-butyl, Haloxyfop-P-methyl, Quizalofop-P-ethyl, Propaquizafop, Cyhalofop-butyl, Clethodim, Sethoxydim, Cycloxydim, Tralkoxydim oder Butroxydim resistent sind;
- und/oder gegen Glyphosat resistent sind,
- und/oder gegen einem oder mehreren die Acetolactatsynthase (ALS) inhibierenden Herbiziden wie beispielsweise ein oder mehrere Sulfonylharnstoff-Herbizide (z.B. Iodosulfuron-methyl, Mesosulfuron-methyl, Tribenuron-methyl, Triasulfuron, Prosulfuron, Sulfosulfuron, Pyrazosulfuron-ethyl, Bensulfuron-methyl, Nicosulfuron, Flazasulfuron, Iofensulfuron, Metsulfuron-methyl, oder jeder andere Sulfonylharnstoff, der im "The Pesticide Manual", 15. Ausgabe (2009) oder 16. Ausgabe (2012), C.D.S. Tomlin, British Crop Protection Council offenbart ist und/oder einem oder mehreren Triazolopyrimidin Herbiziden (z.B. Florasulam, Pyroxsulam oder Penoxsulam) und/oder einem oder mehreren Pyrimidinyl-(thio oder oxy)-benzoat Herbiziden (z.B. Bispyribac-Natrium oder Pyriftalid) und/oder einem oder mehreren Sulfonylaminocarbonyltriazolinon Herbiziden (z.B. Thiencarbazon-methyl, Propoxycarbazone-Natrium oder Flucarbazone-Natrium) und/oder Imidazolinone Herbiziden (z.B. Imazamox) resistent sind.

Spezifische Beispiele für solche gegen ACCase- und/oder ALS-Inhibitoren und/oder Glyphosat-resistente Schadgräser sind unter anderem Alopecurus myosuroides, Apera spicaventi, Avena fatua, Avena sterilis, Brachiaria decumbens, Brachiaria plantaginea, Digitatia horizontalis, Digitaria insularis, Digitaria sanguinalis, Echinochloa colona, Echinochloa crus-galli, Eleusine indica, Lolium multiflorum, Lolium rigidum, Lolium perenne, Phalaris minor, Phalaris paradoxa, Setaria viridis, Setaria faberi oder Setaria glauca.

In einer besonders bevorzugten Ausführungsform der vorliegenden Erfindung können die erfindungsgemäßen Verbindungen der allgemeinen Formel (I) gegen Schadpflanzen eingesetzt werden,
- die gegenüber einem oder mehreren ACCase inhibierenden Herbiziden (z.B. ausgewählt aus der obigen Liste) resistent sind und zwar zumindest teilweise aufgrund von Mutationen (z.B. Substitution) einer oder mehrerer Aminosäuren in der ACCase Targetsite der Schadpflanze (vgl. z.B. S.B. Powles und Qin Yu, "Evolution in Action: Plants Resistant to Herbicides", Annu. Rev. Plant Biol., 2010, 61, S. 317-347); und/oder
- die gegenüber Glyphosat resistent sind, und zwar zumindest teilweise aufgrund von Mutation (z.B. Substitution) einer oder mehrerer Aminosäuren auf der EPSPS Targetsite in dem betreffenenden Unkraut, auf das Glyphosat ausgerichtet ist; und/oder
- die gegenüber einem oder mehreren ALS-inhibierenden Herbiziden (z.B. ausgewählt aus der obigen Liste der ALS-inhibierenden Herbizide) resistent sind und zwar zumindest teilweise aufgrund von Mutationen (z.B. Substitution) einer oder mehrerer Aminosäuren in der ALS Targetsite in dem betreffenden Unkraut (vgl. z.B. S.B. Powles und Qin Yu, "Evolution in Action: Plants Resistant to Herbicides", Annu. Rev. Plant Biol., 2010, 61, S. 317-347); und/oder
- die gegenüber einem oder mehreren ACCase-inhibierenden Herbiziden (z.B. ausgewählt aus der obigen Liste) und/oder gegen Glyphosatund/oder gegen ein oder mehrere ALSinhibierende Herbizide (z.B. ausgewählt aus der obigen Liste) resistent sind und zwar zumindest teilweise durch eine metabolisch bedingte Herbizid-Resistenz, z. B. zumindest teilweise durch einen Cytochrome P450-vermittelten Metabolismus (vgl. z.B. S.B. Powles und Qin Yu, "Evolution in Action: Plants Resistant to Herbicides", Annu. Rev. Plant Biol., 2010, 61, S. 317-347).

Die erfindungsgemäßen Verbindungen zeigen im Vergleich zu den Verbindungen aus dem Stand der Technik, beispielsweise der WO 2015/040114, Verbindung 41.03 (siehe auch die Vergleichsdaten in den Tabellen 9 und 10) überlegene Eigenschaften.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl in Wasser und Wasser in Öl Emulsionen, versprühbare Lösungen, Suspensionskonzentrate (SC), Dispersionen auf Öl oder Wasserbasis, ölmischbare Lösungen, Kapselsuspensionen (CS), Stäubemittel (DP), Beizmittel, Granulate für die Streu und Bodenapplikation, Granulate (GR) in Form von Mikro , Sprüh , Aufzugs und Adsorptionsgranulaten, wasserdispergierbare Granulate (WG), wasserlösliche Granulate (SG), ULV Formulierungen, Mikrokapseln und Wachse. Diese einzelnen Formulierungstypen sind im Prinzip bekannt und werden beispielsweise beschrieben in: Winnacker Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind z.B. Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykol-ethersulfate, Alkansulfonate, Alkylbenzolsulfonate, ligninsulfonsaures Natrium, 2,2' dinaphthylmethan 6,6' disulfonsaures Natrium, dibutylnaphthalin sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium Salze wie Ca Dodecylbenzolsulfonat oder nichtionische Emulgatoren wie Fettsäurepolyglykol-ester, Alkylarylpolyglykolether, Fettalkoholpolyglykolether, Propylenoxid Ethylenoxid
Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser oder Ölbasis sein. Sie können beispielsweise durch Naß Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl in Wasser Emulsionen (EW), lassen sich beispielsweise mittels Rührern, Kolloidmühlen und/oder statischen Mischern unter Verwendung von wässrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschten falls in Mischung mit Düngemitteln - granuliert werden. Wasserdispergierbare Granulate werden in der Regel nach den üblichen Verfahren wie Sprühtrocknung, Wirbelbett Granulierung, Teller Granulierung, Mischung mit Hochgeschwindigkeitsmischern und Extrusion ohne festes Inertmaterial hergestellt.

Zur Herstellung von Teller , Fließbett , Extruder und Sprühgranulate siehe z.B. Verfahren in "Spray Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw Hill, New York 1973, S. 8 57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81 96 und J.D. Freyer, S.A. Evans, "Weed Control Handbook", 5th Ed., Blackwell Scientific Publications, Oxford, 1968, Seiten 101 103.

Die agrochemischen Zubereitungen enthalten in der Regel 0.1 bis 99 Gew. %, insbesondere 0.1 bis 95 Gew. %, erfindungsgemäße Verbindungen.

In Spritzpulvern beträgt die Wirkstoffkonzentration z.B. etwa 10 bis 90 Gew. %, der Rest zu 100 Gew. % besteht aus üblichen Formulierungsbestandteilen. Bei emulgierbaren Konzentraten kann die Wirkstoffkonzentration etwa 1 bis 90, vorzugsweise 5 bis 80 Gew. % betragen. Staubförmige Formulierungen enthalten

1 bis 30 Gew. % Wirkstoff, vorzugsweise meistens 5 bis 20 Gew. % an Wirkstoff, versprühbare Lösungen enthalten etwa 0.05 bis 80, vorzugsweise 2 bis 50 Gew. % Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew. %, vorzugsweise zwischen 10 und 80 Gew. %.

Daneben enthalten die genannten Wirkstofformulierungen gegebenenfalls die jeweils üblichen Haft , Netz , Dispergier , Emulgier , Penetrations , Konservierungs , Frostschutz und Lösungsmittel, Füll , Träger und Farbstoffe, Entschäumer, Verdunstungshemmer und den pH Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachfolgenden Beispiele erläutern zusätzlich die vorliegende Erfindung.

### A. Chemische Beispiele

Beispiel D3: 3-[2,6-Dimethyl-4-(prop-1-in-1-yl)phenyl]-4-hydroxy-7-propoxy-1-azaspiro[4.5]dec-3-en-2-on 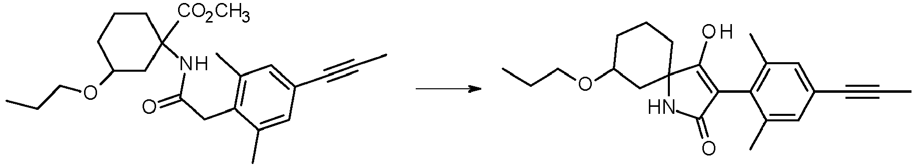 1.50 g (3.75 mmol) Methyl-1-({[2,6-dimethyl-4-(prop-1-in-1-yl)phenyl]-acetyl}amino)-3-propoxycyclohexancarboxylat in 10 ml DMF wurden innerhalb von 30 min bei Raumtemperatur zu einer Lösung von 1.05 g Kalium-t-butylat (9.2 mmol) in 5 ml DMF zugetropft und über Nacht bei dieser Temperatur gerührt. Die Mischung wurde auf Eiswasser gegeben, mit 2N Salzsäure auf pH 1 angesäuert und der ausgefallene Niederschlag abgesaugt. Nach Trocknen erhielt man 1.18 g (86%) der Titelvebindung in Form farbloser Kristalle mit Fp. 219 °C.

### Tabelle 7: Beispiel-Nummern D1-D14

| | | | | | |
|---|---|---|---|---|---|
| In Analogie zu Beispiel D3 sowie gemäß den allgemeinen Angaben zur Herstellung erhält man folgende erfindungsgemäßen Verbindungen. | | | | | |
| 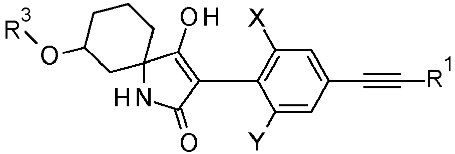 | | | | | |

| **Beispiel Nr.** | **R³** | **X** | **Y** | **R¹** | **¹H-NMR [400 MHz, δ in ppm, d₆-DMSO] oder Schmelzpunkt [°C]** |
|---|---|---|---|---|---|
| D1 | C₂H₅ | C₂H₅ | C₂H₅ | CH₃ | Fp. 235 °C |
| D2 | C₂H₅ | CH₃ | CH₃ | CH₃ | Fp. 285 °C |
| D3 | n-C₃H₇ | CH₃ | CH₃ | CH₃ | Fp. 219 °C |
| D4 | n-C₄H₉ | CH₃ | CH₃ | H | Fp. 135-136 °C |
| D5 | -CH₂CH₂OCH₃ | C₂H₅ | C₂H₅ | CH₃ | δ = 1.01 (mc, 6H), 1.55-1.80 (m, 4H), 2.02 (s, 3H), 2.37 (mc, 4H), 3.25 (s, 3H), 3.41 (mc, 2H), 3.48-3.61 (m, 3H), 7.05 (s, 2H) |
| D6 | -CH₂CH₂OCH₃ | CH₃ | CH₃ | CH₃ | δ = 1.08 (mc, 1H) 1.30 (mc, 1H), 1.58-1.82 (m, 4H), 2.02 (mc, 9H), 3.24 (s, 3H), 3.41 (mc, 2H), 3.52 (mc, 2H), 3.57 (mc, 1H), 7.05 (s, 2H) |
| D7 | -CH₂CH₂OCH₃ | H | C₂H₅ | CH₃ | |
| D8 | -CH₂CH₂OCH₃ | CH₃ | C₂H₅ | CH₃ | δ = 1.00 (mc, 3H), 2.05 (s, 3H), 2.40 (mc, 2H), 3.25 (s, 3H), 3.40 (mc, 2H), 3.47-3.62 (m, 3H), 7.05 (s, 1H), 7.07 (s, 1H) |
| D9 | -CH₂CH₂OCH₃ | CH₃ | C₂H₅ | H | δ = 1.02 (mc, 3H), 1.60-1.88 (m, 5H), 2.05 und 2.08 (je s, Σ3H), 2.43 (mc, 2H), 3.22 (s, 3H), 3.40-3.62 (m, 3H), 4.08 (s, 1H), 7.13 (s, 1H), 7.16 (s, 1H) |
| D10 | -CH₂CH₂OCH₃ | C₂H₅ | C₂H₅ | H | δ = 1.05 (mc, 6H), 2.40 (mc, 4H), 3.24 (s, 3H), 3.41 (mc, 2H), 3.45-3.65 (m, 3H), 4.09 (s, 1H), 7.15 (s, 2H) |
| D11 | n-C₃H₇ | C₂H₅ | CH₃ | CH₃ | δ = 0.86 (t, 3H), 1.02 (t, 3H), 1.09 (mc, 1H), 1.29 (mc, 1H), 1.48 (quint., 2H), 2.02 (s, 3H), 2.40 (mc, 2H), 3.55 (mc, 1H), 7.06 (s, 1H). 7.07 (s, 1H) |
| D12 | n-C₃H₇ | C₂H₅ | C₂H₅ | CH₃ | |
| D13 | n-C₃H₇ | CH₃ | CH₃ | H | δ = 1.31 (mc, 2H), 2.08 (s, 3H), 2.11 (s, 3H), 3.25 (s, 3H), 3.40 (mc, 2H), 3.52 (mc, 2H), 3.59 (mc, 1H), 4.09 (s, 1H), 7.15 (s, 2H) |
| D14 | n-C₃H₇ | CH₃ | C₂H₅ | H | |

Die obengenannte Verbindung D7 fällt nicht unter die Ansprüche und stellt somit eine Referenzverbindung dar. Beispiel P7: 3-(4-Ethinyl-2,6-diethylphenyl)-4-hydroxy-7-(2-methoxyethoxy)-1-azaspiro[4.5]dec-3-en-2-on 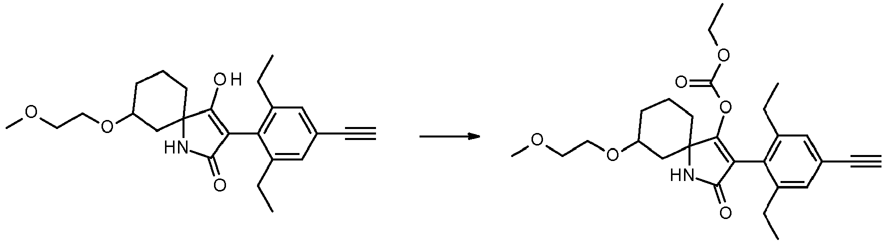 176 mg g (0.44 mmol) 3-(4-Ethinyl-2,6-diethylphenyl)-4-hydroxy-8-methoxy-1-azaspiro[4.5]dec-3-en-2-on wurden mit 0.5 ml Triethylamin in 8 ml Dichlormethan vorgelegt und 10 min bei 40°C gerührt. Anschließend tropfte man 58 mg (0.53 mmol) Chlorameisensäureethylester in 3 ml Dichlormethan langsam zu und ließ anschließend 3 h bei Raumtemperatur rühren. Waschen mit 10 ml Natriumhydrogencarbonat-Lösung und 10 ml Wasser, Trocknen (Magnesiumsulfat) und Abdestillieren des Lösungsmittels wurde das Rohprodukt säulenchromatographisch an Silicagel (Ethylacetat / n-Heptan) gereinigt. Man erhielt so 70 mg (33%) der Titelverbindung als farblosen Feststoff.

**Tabelle 8: Beispiel-Nummern P1-P23**

| | | | | | | |
|---|---|---|---|---|---|---|
| In Analogie zu Beispiel P7 sowie gemäß den allgemeinen Angaben zur Herstellung erhält man folgende erfindungsgemäßen Verbindungen: | | | | | | |
| 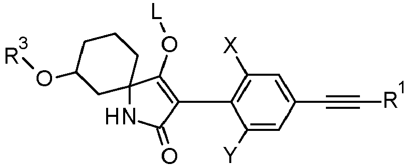 | | | | | | |

| **Beispiel Nr.** | **R³** | **X** | **Y** | **R¹** | **L** | **¹H-NMR [400 MHz, δ in ppm, CDCl₃] oder Schmelzpunkt [°C]** |
|---|---|---|---|---|---|---|
| P1 | C₂H₅ | C₂H₅ | CH₃ | CH₃ | CO₂C₂H₅ | |
| P2 | C₂H₅ | C₂H₅ | C₂H₅ | CH₃ | CO₂C₂H₅ | |
| P3 | C₂H₅ | C₂H₅ | C₂H₅ | CH₃ | COiPr | Fp. 206 °C |
| P4 | C₂H₅ | C₂H₅ | C₂H₅ | CH₃ | CO₂C₂H₅ | Fp. 202 °C |
| P5 | n-C₄H₉ | CH₃ | CH₃ | H | CO₂C₂H₅ | Fp. 173 °C |
| P6 | n-C₄H₉ | CH₃ | CH₃ | H | COiPr | Fp. 208 °C |
| P7 | -CH₂CH₂OCH₃ | C₂H₅ | C₂H₅ | H | CO₂C₂H₅ | δ = 1.15 (mc, 9H), 1.80 (mc, 2H), 1.99 (mc, 2H), 2.20 (mc, 2H), 2.52 (mc, 4H), 3.05 (s, 1H) , 3.38 (s, 3H), 3.46 (mc, 1H), 3.52 (mc, 2H), 4.02 (q, 2H), 7.23 (s, 2H) |
| P8 | -CH₂CH₂OCH₃ | C₂H₅ | C₂H₅ | H | COiPr | δ = 0.98 (d, 3H), 1.18 (mc, 6H), 2.45-2.60 (m, 5H), 3.04 (s, 1H), 3.38 (s, 3H), 3.48 (mc, 1H), 3.51 (mc, 2H), 3.56-3.70 (m, 2H), 7.22 (s, 2H) |
| P9 | -CH₂CH₂OCH₃ | CH₃ | C₂H₅ | H | CO₂C₂H₅ | δ = 1.10-1.19 (mc, 6H), 1.72-1.88 (m, 2H), 1.90-2.05 (m, 2H), 2.50 (mc, 2H), 3.38 (s, 3H), 3.52 (mc, 2H), 4.01 (q, 2H), |
| P10 | -CH₂CH₂OCH₃ | CH₃ | C₂H₅ | H | COiPr | δ = 0.98 (mc, 6H), 1.15 (mc, 3H), 1.90-2.05 (m, 2H), 2.19 und 2.22 (je s, Σ 3H), 2.50 (mc, 3H), 3.02 (s, 1H), 3.39 (s, 3H), 3.40-3.72 (m, 5H) |
| P11 | CH₂CH₂OCH₃ | CH₃ | CH₃ | H | CO₂C₂H₅ | δ = 1.12 (t, 3H), 1.80 (mc, 2H), 1.99 (mc, 2H), 2.19 (s, 3H), 2.21 (s, 3H), 3.02 (s, 1H), 3.38 (s, 3H), 3.48 (mc, 1H), 3.51 (mc, 2H), 3.63 (mc, 2H), 4.02 (q, 2H), 7.22 (s, 1H), 7.24 (s, 1H) |
| P12 | CH₂CH₂OCH₃ | C₂H₅ | C₂H₅ | CH₃ | COiPr | δ = 0.97 (d, 6H), 1.12 (mc, 6H), 2.05 (s, 3H), 2.49 (mc, 2H), 3.38 (s, 3H), 3.45 (mc, 1H), 3.52 (mc, 2H), 3.48-3.70 (m, 2H), 7.12 (s, 2H) |
| P13 | CH₂CH₂OCH₃ | CH₃ | C₂H₅ | CH₃ | CO₂C₂H₅ | δ = 1.10-1.18 (mc, 6H), 2.02 (s, 3H), 2.48 (mc, 2H), 3.38 (s, 3H), 4.02 (q, 2H), 7.10 (mc, 2H) |
| P14 | CH₂CH₂OCH₃ | CH₃ | CH₃ | CH₃ | CO₂iPr | δ = 1.08 (mc, 6H), 2.00 (s, 3H), 2.18 (mc, 6H), 3.47 (s, 3H), 3.43 (mc, 1H), 3.51 mc, 2H), 3.62 (mc, 2H), 4.62 (mc, 1H), 7.07 (s, 2H) |
| P15 | CH₂CH₂OCH₃ | CH₃ | CH₃ | CH₃ | CO₂C₂H₅ | δ = 1.12 (t, 3H), 2.02 (s, 3H), 2.18 (mc, 6H), 3.38 (s, 3H), 3.43 (mc, 1H), 3.51 (mc, 2H), 3.62 (mc, 2H), 4.00 (q, 2H), 7.08 (s, 2H) |
| P16 | CH₂CH₂OCH₃ | CH₃ | CH₃ | CH₃ | CO₂CH₃ | δ = 1.23 (mc, 2H), 1.48 (mc, 2H),, 1.79 (mc, 2H), und 1.99 (mc, 2H), 2.02 (s, 3H), 2.18 (mc, 6H), 3.38 (s, 3H), 3.47 (mc, 1H), 3.51 (mc, 2H), 3.58 (s, 3H), 3.55-3.70 (m, 2H), 7.08 (s, 2H) |
| P17 | CH₂CH₂OCH₃ | CH₃ | CH₃ | H | COiPr | δ = 1.00 (d, 6H), 1.91-2.05 (m, 2H), 2.19 (s, 3H), 2.21 (s, 3H), 2.52 (hept., 1H), 3.01 (s, 1H), 3.38 (s, 3H), 3.45 (mc, 1H), 3.51 (mc, 2H), 3.63 (mc, 2H), 7.18 (s, 2H) |
| P18 | n-C₃H₇ | CH₃ | CH₃ | CH₃ | CO₂CH₃ | |
| P19 | n-C₃H₇ | CH₃ | C₂H₅ | CH₃ | CO₂C₂H₅ | |
| P20 | n-C₃H₇ | CH₃ | C₂H₅ | CH₃ | CO₂C₂H₅ | |
| P21 | n-C₃H₇ | CH₃ | C₂H₅ | CH₃ | COiPr | δ = 0.89-1.02 (m, 6H), 1.12 (mc, 3H), 2.02 (s, 3H), 2.18 und 2.20 (je s, Σ3H), 2.40-2.58 (m, 2H), 3.35-3.49 (m, 2H), 7.09 (s, 1H), 7.11 (s, 1H) |
| P22 | n-C₃H₇ | C₂H₅ | C₂H₅ | CH₃ | CO₂C₂H₅ | |
| P23 | n-C₃H₇ | C₂H₅ | C₂H₅ | CH₃ | COiPr | |

### Herstellungsbeispiele (Ausgangsmaterialien)

Beispiel G24: Methyl-1-({[2-ethyl-6-methyl-4-(prop-1-in-1-yl)phenyl]acetyl}amino)-3 -(2-methoxyethoxy)cyclo hexancarboxylat 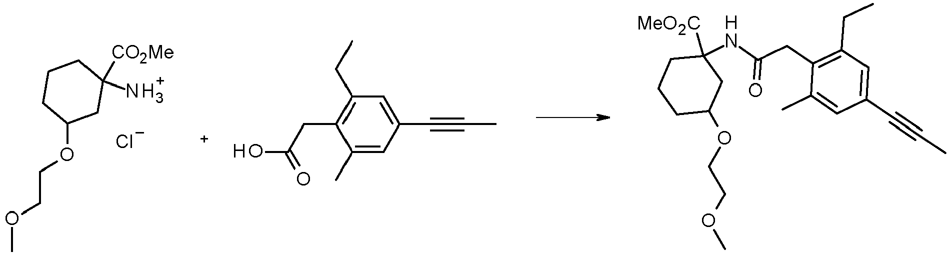 1.00 g (4.6 mmol) 2-Ethyl-6-methyl-4-propinylphenylessigsäure wurden in 25 ml Dichlormethan gelöst und mit einem Tropfen DMF versetzt. Man gab 1.25 g (9.88 mmol) Oxalylchlorid hinzu und erhitzte bis zum Ende der Gasentwicklung unter Rückfluss zum Sieden. Anschließend engte man die Reaktionslösung ein, versetzte noch zweimal mit je 30 ml Dichlormethan und engte erneut ein, um abschließend in 4 ml Dichlormethan aufzunehmen (Lösung 1). 1.33 g (5 mmol) cis-3-methoxyethoxy-1-(methoxycarbonyl)cyclohexanaminiumchlorid sowie 1 g Triethylamin wurden in 20 ml Dichlormethan gelöst und Lösung 1 innerhalb von 90 min zugetropft. Nach 18 h Rühren wurde mit 50 ml Wasser versetzt, die organische Phase abgetrennt, eingeengt und säulenchromatographisch (Silicagel, Gradient Ethylacetat/n-Heptan) gereinigt. Man erhielt 1.71 g (87%) der gewünschten Vorstufe.

**Tabelle 9: Beispiel-Nummern G1-G30**

| | | | | | |
|---|---|---|---|---|---|
| In Analogie zu Beispiel G24 sowie gemäß den allgemeinen Angaben zur Herstellung erhält man folgende Verbindungen | | | | | |
| 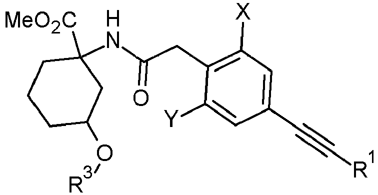 | | | | | |

| **Bsp. Nr.** | **R³** | **X** | **Y** | **R¹** | **¹H-NMR (400 MHz, δ in ppm, CDCl3) bzw. Schmelzpunkt** |
|---|---|---|---|---|---|
| G1 | CH₃ | CH₃ | CH₃ | H | |
| G2 | CH₃ | C₂H₅ | CH₃ | H | |
| G3 | CH₃ | C₂H₅ | C₂H₅ | H | |
| G4 | CH₃ | CH₃ | CH₃ | CH₃ | |
| G5 | CH₃ | C₂H₅ | CH₃ | CH₃ | |
| G6 | CH₃ | C₂H₅ | C₂H₅ | CH₃ | |
| G7 | C₂H₅ | CH₃ | CH₃ | H | |
| G8 | C₂H₅ | CH₃ | C₂H₅ | H | |
| G9 | C₂H₅ | C₂H₅ | C₂H₅ | H | |
| G10 | C₂H₅ | CH₃ | CH₃ | CH₃ | Fp. 158 °C |
| G11 | C₂H₅ | CH₃ | C₂H₅ | CH₃ | |
| G12 | C₂H₅ | C₂H₅ | C₂H₅ | CH₃ | |
| G13 | n-C₃H₇ | CH₃ | CH₃ | H | |
| G14 | n-C₃H₇ | C₂H₅ | CH₃ | H | |
| G15 | n-C₃H₇ | C₂H₅ | C₂H₅ | H | |
| G16 | n-C₃H₇ | CH₃ | CH₃ | CH₃ | Fp. 145 °C |
| G17 | n-C₃H₇ | CH₃ | C₂H₅ | CH₃ | |
| G18 | n-C₃H₇ | C₂H₅ | C₂H₅ | CH₃ | |
| G19 | CH₂CH₂OCH₃ | CH₃ | CH₃ | H | |
| G20 | CH₂CH₂OCH₃ | C₂H₅ | CH₃ | H | |
| G21 | CH₂CH₂OCH₃ | C₂H₅ | C₂H₅ | H | |
| G22 | CH₂CH₂OCH₃ | C₂H₅ | C₂H₅ | CH₃ | δ = 1.22 (mc, 6H), 2.62 (mc, 2H), 3.35 (s, 3H), 3.68 (s, 2H), 7.20 (s, 2H) |
| G23 | CH₂CH₂OCH₃ | CH₃ | CH₃ | CH₃ | δ = 2.01 (s, 3H), 2.33 (s, 6H), 3.47 (s, 3H), 3.53-3.70 (m, 3H), 3.70 (s, 2H), 7.07 (s, 2H) |
| G24 | CH₂CH₂OCH₃ | CH₃ | C₂H₅ | CH₃ | δ = 1.21 (t, 3H), 2.05 (s, 3H), 2.18 (s, 3H), 2.52 (q, 2H), 3.37 (s, 3H), 3.48 (mc, 4H), 3.62 (s, 2H), 7.17 (s, 1H), 7.19 (s, 1H) |
| G25 | n-C₄H₉ | CH₃ | CH₃ | H | Fp. 154-155 °C |
| G26 | n-C₄H₉ | C₂H₅ | CH₃ | H | |
| G27 | n-C₄H₉ | C₂H₅ | C₂H₅ | H | |
| G28 | n-C₄H₉ | CH₃ | CH₃ | CH₃ | |
| G29 | n-C₄H₉ | CH₃ | C₂H₅ | CH₃ | |
| G30 | n-C₄H₉ | C₂H₅ | C₂H₅ | CH₃ | |

### B. Formulierungsbeispiele

a) Ein Stäubemittel wird erhalten, indem man 10 Gew. Teile einer Verbindung der Formel (I) und/oder deren Salze und 90 Gew. Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew. Teile kaolinhaltigen Quarz als Inertstoff, 10 Gewichtsteile ligninsulfonsaures Kalium und 1 Gew. Teil oleoylmethyltaurinsaures Natrium als Netz und Dispergiermittel mischt und in einer Stiftmühle mahlt.
c) Ein in Wasser leicht dispergierbares Dispersionskonzentrat wird erhalten, indem man 20 Gew. Teile einer Verbindung der Formel (I) und/oder deren Salze mit 6 Gew. Teilen Alkylphenolpolyglykolether (®Triton X 207), 3 Gew. Teilen Isotridecanolpolyglykolether (8 EO) und 71 Gew. Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew. Teilen einer Verbindung der Formel (I) und/oder deren Salze, 75 Gew. Teilen Cyclohexanon als Lösungsmittel und 10 Gew. Teilen oxethyliertes Nonylphenol als Emulgator.
e) Ein in Wasser dispergierbares Granulat wird erhalten indem man
   75 Gew. Teile einer Verbindung der Formel (I) und/oder deren Salze,
   10 Gew. Teile ligninsulfonsaures Calcium,
   5 Gew. Teile Natriumlaurylsulfat,
   3 Gew. Teile Polyvinylalkohol und
   7 Gew. Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew. Teile einer Verbindung der Formel (I) und/oder deren Salze,
   5 Gew. Teile 2,2' Dinaphthylmethan 6,6' disulfonsaures Natrium,
   2 Gew. Teile oleoylmethyltaurinsaures Natrium,
   1 Gew. Teil Polyvinylalkohol,
   17 Gew. Teile Calciumcarbonat und
   50 Gew. Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Daten

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 1/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

**Unerwünschte Pflanzen / Weeds:**

| | | | |
|---|---|---|---|
| ALOMY: | *Alopecurus myosuroides* | SETVI: | *Setaria viridis* |
| AMARE: | *Amaranthus retroflexus* | AVEFA: | *Avena fatua* |
| CYPES: | *Cyperus esculentus* | ECHCG: | *Echinochloa crus-galli* |
| LOLMU: | *Lolium multiflorum* | STEME: | *Stellaria media* |
| VERPE: | *Veronica persica* | VIOTR: | *Viola tricolor* |
| POLCO: | *Polygonum convolvulus* | | |

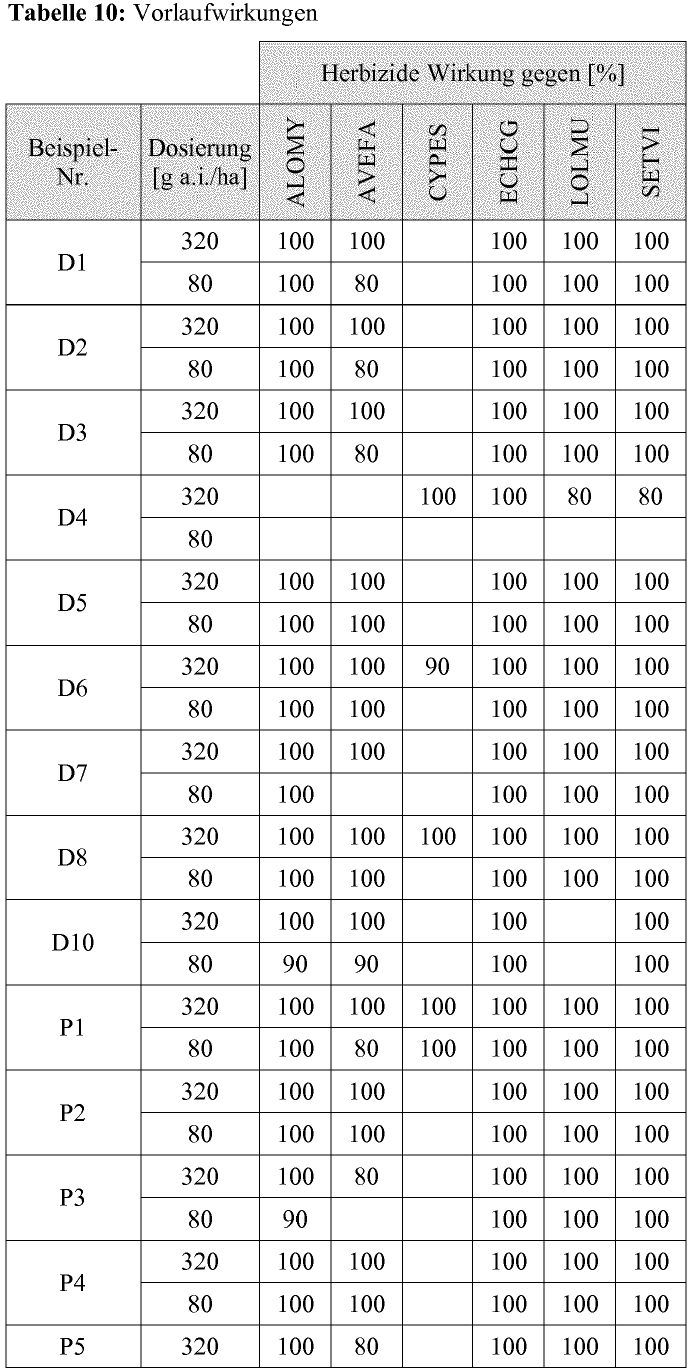

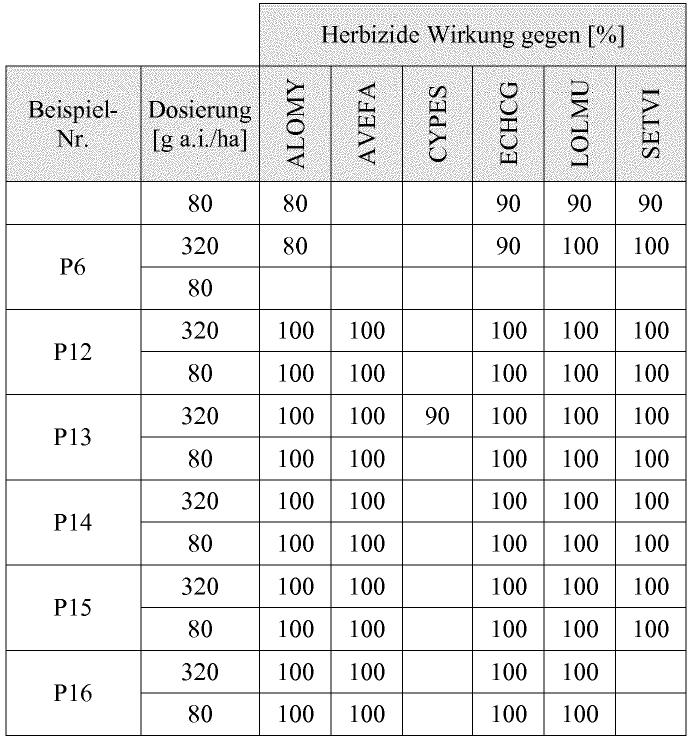

Die obengenannte Verbindung D7 fällt nicht unter die Ansprüche und stellt somit eine Referenzverbindung dar. Wie die Ergebnisse der Tabelle 10 zeigen, weisen die erfindungsgemäßen Verbindungen eine gute herbizide Vorauflaufwirksamkeit gegen ein breites Spektrum von Ungräsern und Unkräutern auf. Beispielsweise zeigen die Verbindungen D1-D6, D8, D10, P1-P6 und P12-P16 bei einer Aufwandmenge von 320 g a.i./ha jeweils eine 80 - 100%-ige Wirkung gegen *Alopecurus myosuroides, Avena fatua, Echinochloa crus-galli, Lolium multiflorum* und *Setaria viridis.* Die erfindungsgemäßen Verbindungen eignen sich deshalb im Vorauflaufverfahren zur Bekämpfung von unerwünschtem Pflanzenwuchs.

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 1/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

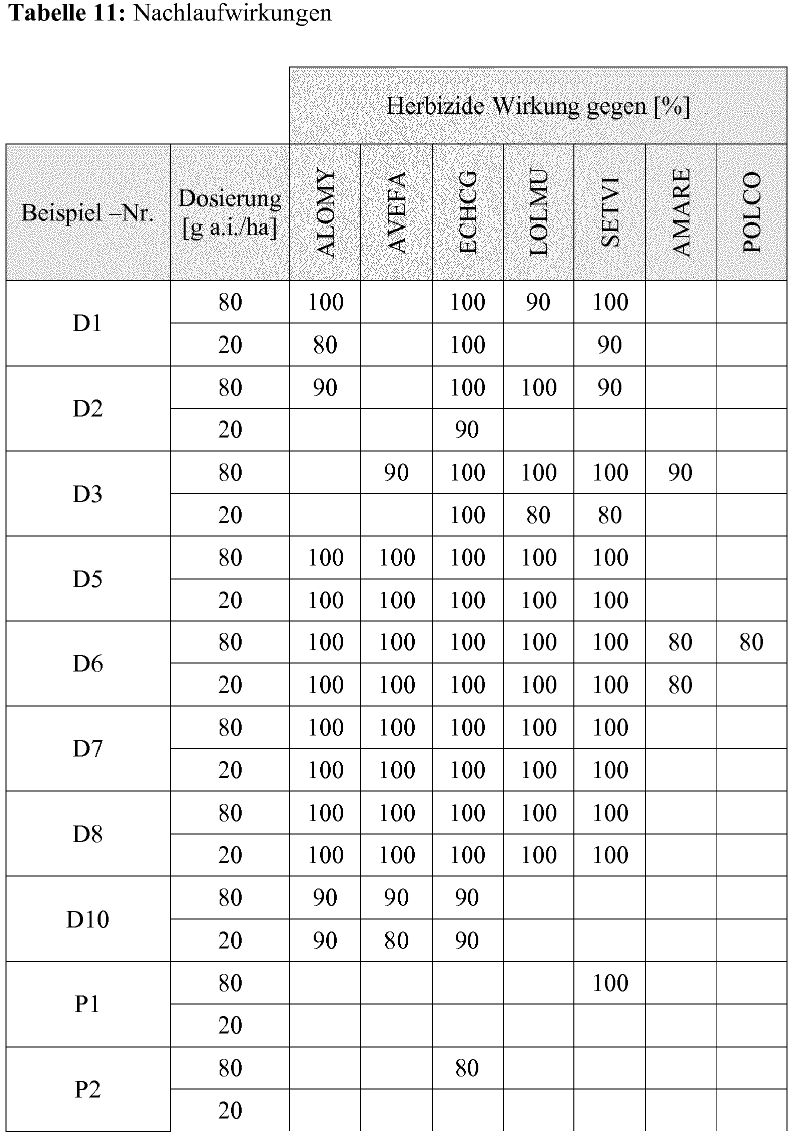

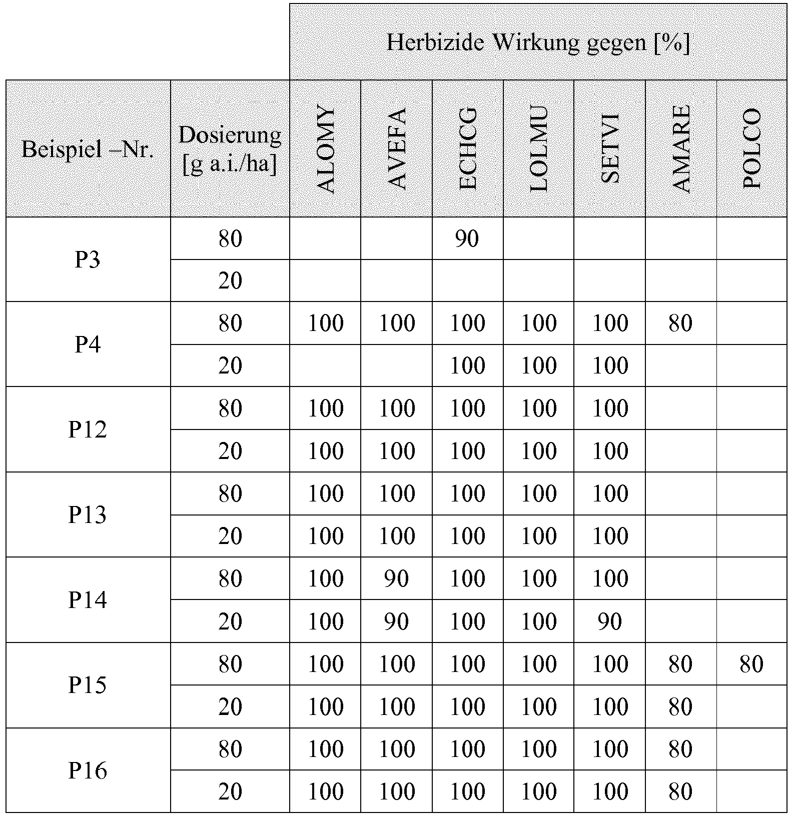

Die obengenannte Verbindung D7 fällt nicht unter die Ansprüche und stellt somit eine Referenzverbindung dar. Wie die Ergebnisse der Tabelle 11 zeigen, weisen die erfindungsgemäßen Verbindungen eine gute herbizide Nachauflaufwirksamkeit gegen ein breites Spektrum von Ungräsern und Unkräutern auf. Beispielsweise zeigen die Verbindungen D1-D6, D8, D10, P1-P4 und P12-P16 und bei einer Aufwandmenge von 80 g/ha jeweils eine 80 - 100%-ige Wirkung gegen *Alopecurus myosuroides, Avena fatua, Echinochloa crus-galli, Lolium multiflorum* und *Setaria viridis.* Die erfindungsgemäßen Verbindungen eignen sich deshalb im Nachauflaufverfahren zur Bekämpfung von unerwünschtem Pflanzenwuchs.

## Patentansprüche

1. Alkinyl-substituierte-N-Phenylpyrrolidin-2,4-dione der allgemeinen Formel (I) 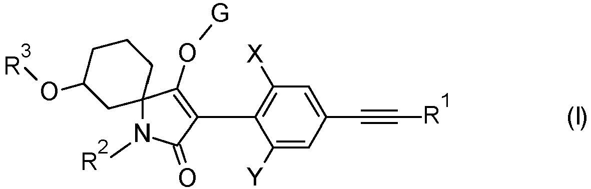 oder ein agrochemisch akzeptables Salz davon, wobei
X = C₁-C₄-Alkyl, C₁-C₄-Haloalkyl oder C₃-C₆-Cycloalkyl,
Y = C₁-C₄-Alkyl oder C₃-C₆-Cycloalkyl,
R¹ = Wasserstoff, C₁-C₆-Alkyl oder C₃-C₆-Cycloalkyl,
R² = Wasserstoff oder Methyl,
R³ = C₁-C₆-Alkyl oder C₁-C₆-Alkoxy-C₂-C₆-alkyl,
G = Wasserstoff, eine abspaltbare Gruppe L oder ein Kation E, wobei L = einer der folgenden Reste 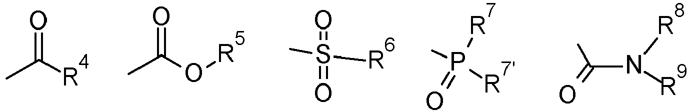
worin
R⁴ = C₁-C₄-Alkyl oder C₁-C₃-Alkoxy-C₁-C₄-Alkyl,
R⁵ = C₁-C₄-Alkyl,
R⁶ = C₁-C₄-Alkyl, ein unsubstituiertes Phenyl oder ein einfach oder mehrfach mit Halogen, C₁-C₄-Alkyl, C₁-C₄-Haloalkyl, C₁-C₄-Alkoxy, C₁-C₄-Haloalkoxy, Nitro oder Cyano substituiertes Phenyl,
R⁷, R^{7'} = unabhängig voneinander Methoxy oder Ethoxy,
R⁸ und R⁹ = jeweils unabhängig voneinander Methyl, Ethyl, Phenyl oder gemeinsam einen gesättigten 5-, 6- oder 7-gliedrigen Ring bilden, oder gemeinsam einen gesättigten 5-, 6-, oder 7-gliedrigen Heterozyklus mit einem Sauerstoff- oder Schwefelatom bilden,
E = ein Alkalimetallion, ein Ionenäquivalent eines Erdalkalimetalls, ein Ionenäquivalent Aluminium, ein Ionenäquivalent eines Übergangsmetalls, ein Magnesium-Halogen-Kation, oder ein Ammoniumion, bei dem gegebenenfalls ein, zwei, drei oder alle vier Wasserstoffatome durch gleiche oder verschiedene Reste aus den Gruppen Wasserstoff, C₁-C₅-Alkyl, C₁-C₅Alkoxy oder C₃-C₇-Cycloalkyl, die jeweils ein- oder mehrfach mit Fluor, Chlor, Brom, Cyano, Hydroxy substituiert oder durch ein- oder mehrere Sauerstoff- oder Schwefelatome unterbrochen sein können, oder
ein cyclisches sekundäres oder tertiäres aliphatisches oder heteroaliphatisches Ammoniumion, beispielsweise Morpholinium, Thiomorpholinium, Piperidinium, Pyrrolidinium, oder jeweils protoniertes 1,4-Diazabicyclo[1.1.2]octane (DABCO) oder 1,5-Diazabicyclo[4.3.0]undec-7-en (DBU), oder
ein heterocyclisches Ammoniumkation, beispielsweise jeweils protoniertes Pyridin, 2-Methylpyridin, 3-Methylpyridin, 4-Methylpyridin, 2,4-Dimethylpyridin, 2,5-Di-methylpyridin, 2,6-Dimethylpyridin, 5-Ethyl-2-methylpyridin, Pyrrol, Imidazol, Chinolin, Chinoxalin, 1,2-Dimethylimidazol, 1,3-Dimethylimidazolium-methylsulfat, steht, oder ein Sulfoniumion ist.

2. Verbindung gemäß Anspruch 1, wobei
X = C₁-C₄-Alkyl oder C₃-C₆-Cycloalkyl,
Y = C₁-C₄-Alkyl oder C₃-C₆-Cycloalkyl,
R¹ = Wasserstoff, Methyl, Ethyl, Isopropyl oder Cyclopropyl,
R² = Wasserstoff oder Methyl,
R³ = C₁-C₆-Alkyl oder C₁-C₄-Alkoxy-C₂-C₄-alkyl,
G = Wasserstoff, eine abspaltbare Gruppe L oder ein Kation E, worin L = einer der folgenden Reste 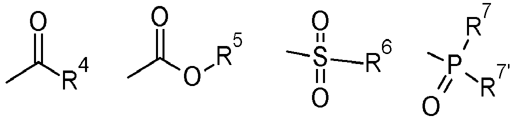
worin
R⁴ = C₁-C₄-Alkyl,
R⁵ = C₁-C₄-Alkyl,
R⁶ = C₁-C₄-Alkyl, ein unsubstituiertes Phenyl oder ein mit Halogen, C₁-C₄-Alkyl oder C₁-C₄-Alkoxy substituiertes Phenyl,
R⁷, R^{7'} = unabhängig voneinander Methoxy oder Ethoxy,
E = ein Alkalimetallion, ein Ionenäquivalent eines Erdalkalimetalls, ein Ionenäquivalent Aluminium oder ein Ionenäquivalent eines Übergangsmetalls, oder
ein Ammoniumion, bei dem gegebenenfalls ein, zwei, drei oder alle vier Wasserstoffatome durch gleiche oder verschiedene Reste aus den Gruppen Wasserstoff oder C₁-C₅-Alkyl, oder ein tertiäres aliphatisches oder heteroaliphatisches Ammoniumion, oder ein heterocyclisches Ammoniumkation, beispielsweise jeweils protoniertes Pyridin, Chinolin, Chinoxalin, 1,2-Dimethylimidazol, 1,3-Dimethylimidazolium-methylsulfat, steht, oder
ein Sulfoniumion ist.

3. Verbindungen gemäß einem der Ansprüche 1 oder 2, wobei R¹ = Methyl, Ethyl, Isopropyl oder Cyclopropyl.

4. Verbindung gemäß einem der v Ansprüche 1 bis 3, wobei R² = Wasserstoff.

5. Verbindung gemäß einem der Ansprüche 1 bis 4, wobei R³ C₁-C₄-Alkyl oder C₁-C₃-Alkoxy-C₂-C₄-alkyl.

6. Verbindung gemäß einem der Ansprüche 1 bis 5, wobei X = Methyl, Ethyl oder Cyclopropyl und Y = Methyl oder Ethyl.

7. Verbindung gemäß einem der vorherigen Ansprüche 1 bis 6, wobei G = Wasserstoff, eine abspaltbare Gruppe L oder ein Kation E, bedeutet, worin
L = einer der folgenden Reste 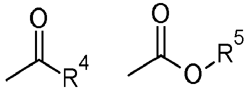 mit
R⁴ = Methyl, Ethyl oder Isopropyl,
R⁵ = Methyl oder Ethyl, und
E = ein Natrium-, Kalium-, Trimethylammonium-, Pyridinium-, Chinolinium- oder Trimethylsulfonium-Kation oder ein Ionenäquivalent Calcium- oder Magnesium.

8. Verbindung gemäß einem der vorherigen Ansprüche 1 bis 7, wobei X = Methyl, Ethyl oder Cyclopropyl, Y = Methyl oder Ethyl, und R² = Wasserstoff.

9. Herbizide Zusammensetzung enthaltend eine Verbindung der allgemeinen Formel (I) gemäß einem der Ansprüche 1 bis 8 oder ein agrochemisch akzeptables Salz davon, und optional einen agrochemisch akzeptabler Träger, Verdünnungsmittel und/oder Lösungsmittel.

10. Herbizide Zusammensetzung gemäß Anspruch 9, enthaltend mindestens einen weiteren agrochemisch wirksamen Stoff aus der Gruppe der Insektizide, Akarizide, Herbizide, Fungizide, Safener und Wachstumsregulatoren.

11. Herbizide Zusammensetzung nach Anspruch 10 enthaltend einen Safener.

12. Herbizide Zusammensetzung nach einem der Ansprüche 9 bis 11, enthaltend ein weiteres Herbizid.

13. Verfahren zur Bekämpfung von unerwünschtem Pflanzenbewuchs, wobei die Verbindung gemäß einem der Ansprüche 1 bis 8, auf die zu bekämpfende Pflanze, Pflanzenteile, Pflanzensamen oder die Fläche, auf der der unerwünschte Pflanzenbewuchs stattfindet, appliziert wird.

14. Verfahren gemäß Anspruch 13, wobei der unerwünschte Pflanzenbewuchs ausgewählt ist aus grasartigen monocotyledonen Unkräutern.

15. Verfahren gemäß Anspruch 13 oder 14, wobei der Pflanzenbewuchs von resistenten Gräsern in Nutzpflanzen bekämpft wird, und wobei die herbizide Zusammensetzung gemäß den Ansprüchen 1 bis 8 auf das zu bekämpfende Unkraut aufgebracht wird.

16. Verfahren gemäß Anspruch 15, wobei die Nutzpflanze ausgewählt ist aus Weizen, Gerste, Roggen, Hafer, Reis, Zuckerrübe, Soja, Raps, Sonnenblume und Mais.

17. Verwendung von Verbindungen der Formel (I) oder ein agrochemisch akzeptables Salz davon gemäß den Ansprüchen 1 bis 8 zur Bekämpfung von Schadpflanzen.

18. Verwendung nach Anspruch 17, **dadurch gekennzeichnet, dass** die Verbindung der Formel (I) oder ein agrochemisch akzeptables Salz davon zur Bekämpfung Schadpflanzen in Kulturen von Nutzpflanzen eingesetzt wird.

19. Verwendung nach Anspruch 18, **dadurch gekennzeichnet, dass** die die Nutzpflanzen transgene Nutzpflanzen sind.

## Claims

1. Alkynyl-substituted N-phenylpyrrolidine-2,4-diones of the general formula (I) 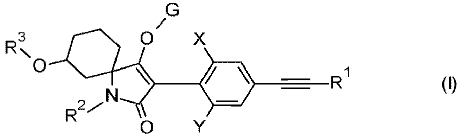 or an agrochemically acceptable salt thereof, where
X = C₁-C₄-alkyl, C₁-C₄-haloalkyl or C₃-C₆-cycloalkyl,
Y = C₁-C₄-alkyl or C₃-C₆-cycloalkyl,
R¹ = hydrogen, C₁-C₆-alkyl or C₃-C₆-cycloalkyl,
R² = hydrogen or methyl,
R³ = C₁-C₆-alkyl or C₁-C₆-alkoxy-C₂-C₆-alkyl,
G = hydrogen, a cleavable group L or a cation E, where L = one of the following radicals 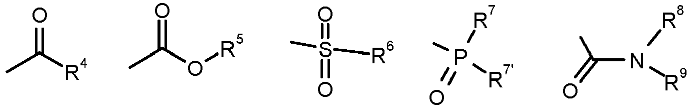
in which
R⁴ = C₁-C₄-alkyl or C₁-C₃-alkoxy-C₁-C₄-alkyl,
R⁵ = C₁-C₄-alkyl,
R⁶ = C₁-C₄-alkyl, an unsubstituted phenyl or a phenyl substituted one or more times with halogen, C₁-C₄-alkyl, C₁-C₄-haloalkyl, C₁-C₄-alkoxy, C₁-C₄-haloalkoxy, nitro or cyano,
R⁷, R^{7'} = independently of one another methoxy or ethoxy,
R⁸ and R⁹ = in each case independently of one another methyl, ethyl, phenyl or together form a saturated 5-, 6- or 7-membered ring, or together form a saturated 5-, 6- or 7-membered heterocycle with an oxygen or sulfur atom,
E = an alkali metal ion, an ion equivalent of an alkaline earth metal, an ion equivalent of aluminium, an ion equivalent of a transition metal, a magnesium halogen cation, or
an ammonium ion, in which optionally one, two, three or all four hydrogen atoms by identical or different radicals from the groups hydrogen, C₁-C₅-alkyl, C₁-C₅-alkoxy or C₃-C₇-cycloalkyl, which can in each case be substituted one or more times with fluorine, chlorine, bromine, cyano, hydroxy or be interrupted by one or more oxygen or sulfur atoms, or
a cyclic secondary or tertiary aliphatic or heteroaliphatic ammonium ion, for example morpholinium, thiomorpholinium, piperidinium, pyrrolidinium, or in each case protonated 1,4-diazabicyclo[1.1.2]octanes (DABCO) or 1,5-diazabicyclo[4.3.0]undec-7-ene (DBU), or
a heterocyclic ammonium cation, for example in each case protonated pyridine, 2-methylpyridine, 3-methylpyridine, 4-methylpyridine, 2,4-dimethylpyridine, 2,5-dimethylpyridine, 2,6-dimethylpyridine, 5-ethyl-2-methylpyridine, pyrrole, imidazole, quinoline, quinoxaline, 1,2-dimethylimidazole, 1,3-dimethylimidazolium methyl sulfate, or
is a sulfonium ion.

2. Compound according to Claim 1, where
X = C₁-C₄-alkyl or C₃-C₆-cycloalkyl,
Y = C₁-C₄-alkyl or C₃-C₆-cycloalkyl,
R¹ = hydrogen, methyl, ethyl, isopropyl or cyclopropyl,
R² = hydrogen or methyl,
R³ = C₁-C₆-alkyl or C₁-C₄-alkoxy-C₂-C₄-alkyl,
G = hydrogen, a cleavable group L or a cation E in which L = one of the following radicals 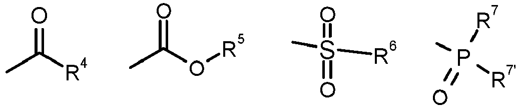
in which
R⁴ = C₁-C₄-alkyl,
R⁵ = C₁-C₄-alkyl,
R⁶ = C₁-C₄-alkyl, an unsubstituted phenyl or a phenyl substituted by halogen, C₁-C₄-alkyl or C₁-C₄-alkoxy,
R⁷, R^{7'} = independently of each other methoxy or ethoxy,
E = an alkali metal ion, an ion equivalent of an alkaline earth metal, an ion equivalent of aluminum or an ion equivalent of a transition metal, or an ammonium ion, in which optionally one, two, three or all four hydrogen atoms by identical or different radicals from the groups hydrogen or C₁-C₅-alkyl, or a tertiary aliphatic or heteroaliphatic ammonium ion, or a heterocyclic ammonium cation, for example in each case protonated pyridine, quinoline, quinoxaline, 1,2-dimethylimidazole, 1,3-dimethylimidazolium methylsulfate, or is a sulfonium ion.

3. Compounds according to either of Claims 1 and 2, where R¹ = methyl, ethyl, isopropyl or cyclopropyl.

4. Compound according to one of Claims 1 to 3, where R² = hydrogen.

5. Compound according to one of Claims 1 to 4, where R³ = C₁-C₄-alkyl or C₁-C₃-alkoxy-C₂-C₄-alkyl.

6. Compound according to one of Claims 1 to 5, where X = methyl, ethyl or cyclopropyl, and Y = methyl or ethyl.

7. Compound according to one of the preceding Claims 1 to 6, where G = hydrogen, a cleavable group L or a cation E in which
L = one of the following radicals 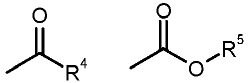 in which R⁴ = methyl, ethyl, or isopropyl,
R⁵ = methyl or ethyl, and
E = a sodium, potassium, trimethylammonium, pyridinium, quinolinium or trimethylsulfonium cation or an ion equivalent of calcium or magnesium.

8. Compound according to one of the preceding Claims 1 to 7, where X = methyl, ethyl or cyclopropyl, Y = methyl or ethyl, and R² = hydrogen.

9. Herbicidal composition comprising a compound of the general formula (I) according to one of Claims 1 to 8 or an agrochemically acceptable salt thereof, and optionally an agrochemically acceptable carrier, diluent and/or solvent.

10. Herbicidal composition according to Claim 9, comprising at least one further agrochemically active substance from the group of insecticides, acaricides, herbicides, fungicides, safeners and growth regulators.

11. Herbicidal composition according to Claim 10, comprising a safener.

12. Herbicidal composition according to one of Claims 9 to 11, comprising a further herbicide.

13. Method of controlling undesired plant growth, where the compound according to one of Claims 1 to 8 is applied to the plant to be controlled, plant parts, plant seeds or the area on which the undesired plant growth takes place.

14. Method according to Claim 13, where the undesired plant growth is selected from grasslike monocotyledonous weeds.

15. Method according to Claim 13 or 14, where the plant growth of resistant grasses in useful plants is controlled, and where the herbicidal composition according to Claims 1 to 8 is applied to the weed to be controlled.

16. Method according to Claim 15, where the useful plant is selected from wheat, barley, rye, oats, rice, sugar cane, soybean, rapeseed, sunflower and corn.

17. Use of compounds of the formula (I) or an agrochemically acceptable salt thereof according to Claims 1 to 8 for controlling harmful plants.

18. Use according to Claim 17, **characterized in that** the compound of the formula (I) or an agrochemically acceptable salt thereof is used for controlling harmful plants in crops of useful plants.

19. Use according to Claim 18, **characterized in that** the useful plants are transgenic useful plants.

## Revendications

1. N-phénylpyrrolidine-2,4-diones à substitution alcynyle de la formule générale (I) : 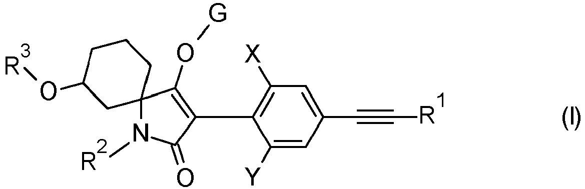 ou un sel agrochimiquement acceptable de celles-ci, dans laquelle
X = alkyle en C₁-C₄, haloalkyle en C₁-C₄ ou cycloalkyle en C₃-C₆,
Y = alkyle en C₁-C₄ ou cycloalkyle en C₃-C₆,
R¹ = hydrogène, alkyle en C₁-C₆ ou cycloalkyle en C₃-C₆,
R² = hydrogène ou méthyle,
R³ = alkyle en C₁-C₆ ou alcoxy en C₁-C₆-alkyle en C₂-C₆,
G = hydrogène, un groupe clivable L ou un cation E, où L = un des radicaux suivants : 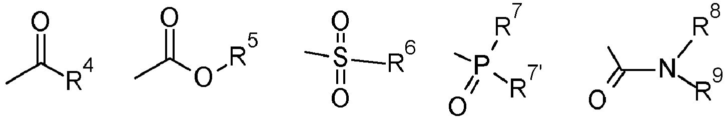
dans lesquels
R⁴ = alkyle en C₁-C₄ ou alcoxy en C₁-C₃-alkyle en C₁-C₄,
R⁵ = alkyle en C₁-C₄,
R⁶ = alkyle en C₁-C₄, un phényle non substitué ou un phényle substitué une fois ou plusieurs fois avec halogène, alkyle en C₁-C₄, haloalkyle en C₁-C₄, alcoxy en C₁-C₄, haloalcoxy en C₁-C₄, nitro ou cyano,
R⁷, R^{7'} = indépendamment l'un de l'autre méthoxy ou éthoxy,
R⁸ et R⁹= chacun indépendamment l'un de l'autre méthyle, éthyle, phényle ou forment ensemble un cycle saturé à 5, 6 ou 7 chaînons, ou forment ensemble un hétérocycle saturé à 5, 6 ou 7 chaînons comprenant un atome d'oxygène ou de soufre,
E = un ion de métal alcalin, un équivalent ionique d'un métal alcalino-terreux, un équivalent ionique d'aluminium, un équivalent ionique d'un métal de transition, un cation magnésium-halogène, ou
un ion ammonium, dans lequel un, deux, trois ou les quatre atomes d'hydrogène sont éventuellement par des radicaux identiques ou différents du groupe constitué par hydrogène, alkyle en C₁-C₅, alcoxy en C₁-C₅ ou cycloalkyle en C₃-C₇, qui peuvent chacun être substitués une ou plusieurs fois avec fluor, chlore, brome, cyano, hydroxy ou interrompus par un ou plusieurs atomes d'oxygène ou de soufre, ou
un ion ammonium cyclique, secondaire ou tertiaire, aliphatique ou hétéroaliphatique, par exemple morpholinium, thiomorpholinium, pipéridinium, pyrrolidinium, ou des 1,4-diazabicyclo[1.1.2]octanes (DABCO) ou du 1,5-diazabicyclo[4.3.0]undéc-7-ène (DBU), chacun protoné, ou
un cation ammonium hétérocyclique, par exemple pyridine, 2-méthylpyridine, 3-méthylpyridine, 4-méthylpyridine, 2,4-diméthylpyridine, 2,5-diméthylpyridine, 2,6-diméthylpyridine, 5-éthyl-2-méthylpyridine, pyrrole, imidazole, quinoline, quinoxaline, 1,2-diméthylimidazole, 1,3-diméthylimidazolium-méthylsulfate, chacun protoné, ou
est un ion sulfonium.

2. Composé selon la revendication 1, dans lequel
X = alkyle en C₁-C₄ ou cycloalkyle en C₃-C₆,
Y = alkyle en C₁-C₄ ou cycloalkyle en C₃-C₆,
R¹ = hydrogène, méthyle, éthyle, isopropyle ou cyclopropyle,
R² = hydrogène ou méthyle,
R³ = alkyle en C₁-C₆ ou alcoxy en C₁-C₄-alkyle en C₂-C₄,
G = hydrogène, un groupe clivable L ou un cation E, où L = un des radicaux suivants : 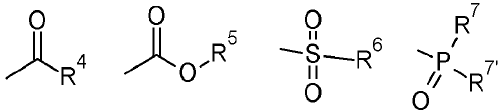
dans lesquels R⁴ = alkyle en C₁-C₄, R⁵ = alkyle en C₁-C₄,
R⁶ = alkyle en C₁-C₄, un phényle non substitué ou un phényle substitué avec halogène, alkyle en C₁-C₄ ou alcoxy en C₁-C₄,
R⁷, R^{7'} = indépendamment l'un de l'autre méthoxy ou éthoxy,
E = un ion de métal alcalin, un équivalent ionique d'un métal alcalino-terreux, un équivalent ionique d'aluminium ou un équivalent ionique d'un métal de transition, ou
un ion ammonium, dans lequel un, deux, trois ou les quatre atomes d'hydrogène sont éventuellement par des radicaux identiques ou différents du groupe constitué par hydrogène ou alkyle en C₁-C₅, ou un ion ammonium tertiaire, aliphatique ou hétéroaliphatique, ou un cation ammonium hétérocyclique, par exemple pyridine, quinoline, quinoxaline, 1,2-diméthylimidazole, 1,3-diméthylimidazolium-méthylsulfate, chacun protoné, ou
est un ion sulfonium.

3. Composés selon l'une quelconque des revendications 1 ou 2, dans lesquels R¹ = méthyle, éthyle, isopropyle ou cyclopropyle.

4. Composé selon l'une quelconque des revendications 1 à 3, dans lequel R² = hydrogène.

5. Composé selon l'une quelconque des revendications 1 à 4, dans lequel R³ représente alkyle en C₁-C₄ ou alcoxy en C₁₋C₃-alkyle en C₂-C₄.

6. Composé selon l'une quelconque des revendications 1 à 5, dans lequel X = méthyle, éthyle ou cyclopropyle, et Y = méthyle ou éthyle.

7. Composé selon l'une quelconque des revendications 1 à 6 précédentes, dans lequel G = hydrogène, un groupe clivable L ou un cation E, où
L = un des radicaux suivants : 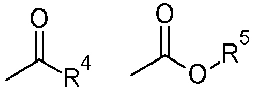 avec
R⁴ = méthyle, éthyle ou isopropyle,
R⁵ = méthyle ou éthyle, et
E = un cation sodium, potassium, triméthylammonium, pyridinium, quinolinium ou triméthylsulfonium ou un équivalent ionique de calcium ou de magnésium.

8. Composé selon l'une quelconque des revendications 1 à 7 précédentes, dans lequel X = méthyle, éthyle ou cyclopropyle, Y = méthyle ou éthyle, et R² = hydrogène.

9. Composition herbicide contenant un composé de la formule générale (I) selon l'une quelconque des revendications 1 à 8 ou un sel agrochimiquement acceptable de celui-ci, et éventuellement un véhicule, diluant et/ou solvant agrochimiquement acceptable.

10. Composition herbicide selon la revendication 9, contenant au moins une substance à activité agrochimique supplémentaire du groupe constitué par les insecticides, les acaricides, les herbicides, les fongicides, les agents protecteurs et les régulateurs de croissance.

11. Composition herbicide selon la revendication 10, contenant un agent protecteur.

12. Composition herbicide selon l'une quelconque des revendications 9 à 11, contenant un herbicide supplémentaire.

13. Procédé pour combattre une végétation indésirable, dans lequel le composé selon l'une quelconque des revendications 1 à 8 est appliqué sur les plantes à combattre, des parties des plantes, des graines des plantes ou la surface sur laquelle la végétation indésirable se trouve.

14. Procédé selon la revendication 13, dans lequel la végétation indésirable est choisie parmi les mauvaises herbes monocotylédones graminées.

15. Procédé selon la revendication 13 ou 14, dans lequel la végétation de graminées résistantes dans des plantes utiles est combattue, et dans lequel la composition herbicide selon les revendications 1 à 8 est appliquée sur la mauvaise herbe à combattre.

16. Procédé selon la revendication 15, dans lequel la plante utile est choisie parmi le blé, l'orge, le seigle, l'avoine, le riz, la betterave sucrière, le soja, le colza, le tournesol et le maïs.

17. Utilisation de composés de la formule (I) ou d'un sel agrochimiquement acceptable de ceux-ci selon les revendications 1 à 8 pour combattre des plantes nocives.

18. Utilisation selon la revendication 17, **caractérisée en ce que** le composé de la formule (I) ou un sel agrochimiquement acceptable de celui-ci est utilisé pour combattre des plantes nocives dans des cultures de plantes utiles.

19. Utilisation selon la revendication 18, **caractérisée en ce que** les plantes utiles sont des plantes utiles transgéniques.
